# EUROPEAN PATENT APPLICATION

(11) **EP 2 813 138 A1**
(43) Date of publication of application: **17.12.2014**
(21) Application number: 13746154.7
(22) Date of filing: 06.02.2013
(51) Int. Cl.: A01G 7/00, A01G 1/00, A01G 31/00, A23L 1/30

(54) **TECHNIQUE AND METHOD FOR PRODUCING FUNCTIONAL MATERIAL ORIGINATED FROM ICE PLANT, AND FUNCTIONAL COMPONENT**

(30) Priority: 06.02.2012 JP 2012023050
(71) Applicant: Tsujiko Co., Ltd., Koka-shi, Shiga 528-0057 (JP)
(72) Inventor: CHE, Fang-sik, Nagahama-shi Shiga 526-0829 (JP); TSUJI, Akihisa, Koka-shi Shiga 528-0027 (JP)
(74) Representative: Müller, Christian Stefan Gerd
(86) International application number: PCT/JP2013/052702
(87) International publication number: WO 2013/118760

(57) **Abstract**

The present invention provides a method for producing an ice plant having increased contents of pinitol, β-carotene, vitamin K and proline, said method being characterized by adding a stress to an ice plant during the cultivation of the ice plant; a method for increasing the contents of pinitol, β-carotene, vitamin K and proline" which are functional components contained in an ice plant, said method being characterized by adding a stress to the ice plant during the cultivation of the ice plant; and others.

## Description

### Technical Field

The present invention relates to a method of producing a functional material derived from an ice plant, and the functional material. The present invention particularly relates to a method of increasing functional components in an ice plant, and an ice plant having increased contents of functional components.

### Background Art

Ice plant (scientific name: Mesembryanthemum crystallium; English name: common ice plant) is an annual plant belonging to the Aizoaseae family, Lampranthus genus, and a halophyte plant native to the Namib Desert in South Africa. The leaves and the tip portions of offshoots of ice plant are used as food to be eaten raw or to be cooked. Ice plant is a vegetable with a unique texture and a salty taste. Currently, ice plant is marketed as the trade names "Tsuburina", "Barafu", "Puttina", etc.

When ice plant receives a certain level of salt stress or drying stress, it can switch from C3 photosynthesis to CAM (Crassulacean acid metabolism) photosynthesis (hereinafter, also referred to as "CAM-switching"), and thereby can continue to grow without growth failure (see, for example, non-patent literature 1 and non-patent literature 2). Therefore, ice plant draws attention as a stress-induced and controlled C3/CAM switching-type plant that can switch between C3 photosynthesis, which general plants carry out, and CAM photosynthesis, which plants in dry lands carry out.

The CAM photosynthesis is one type of photosynthesis found frequently in succulent plants living in desert, etc. and epiphytes living in water-stressed environments like desert, and is characterized by uptake of carbon dioxide during the night and reduction of the carbon dioxide during the day. Such plants are referred to as "CAM plants". The CAM plants open their stomata to take up carbon dioxide during the night which is cool and close the stomata to minimize the loss of water due to transpiration during the day. Ice plant can prevent moisture decrease in the plant body due to dryness by switching to CAM photosynthesis, and can also tolerate adverse conditions by releasing absorbed salts into saclike transparent cells formed on the stem and the leaf surfaces, called "bladder cells". The existence of the "bladder cells" provides ice plant with a unique texture and a unique taste.

Ice plant is rich in various functional components such as minerals (sodium, potassium, calcium, manganese, magnesium, zinc, etc.); vitamin A and analogs thereof, for example β-carotene and retinol; vitamin K; pantothenic acid; inositol and analogs thereof (ononitol, myo-inositol, pinitol, etc.); and organic acids (malic acid, citric acid, etc.), and therefore it is expected to have lifestyle disease prevention effect, blood glucose level-lowering effect, antioxidant effect, anti-aging effect, etc. are expected (see, for example, non-patent literature 3). These functional components in ice plant are known to accumulate in response to salt stress and drying stress (see, for example, non-patent literature 4). However, the detail has not yet been known, and for example, it has not yet been known which component is produced in an increased level under what kind of conditions.

On the other hand, as dietary habit is changed to Western-style, the demand of supplements such as nutritional supplements and health supplements is increasing worldwide, and novel supplement materials are needed. Based on in particular customer's needs, naturally-derived functional materials rather than synthetic materials are desired, and therefore, plant-derived functional components (phytochemicals) are attracting attention.

### Citation List

### Non-patent Literatures

Non-patent Literature 1: Bohnert et al., J Plant Growth Regul, 2000, 19: 334-346
Non-patent Literature 2: Gendai Nougyou 2009. 2, Pages 77-79, Rural Culture Association
Non-patent Literature 3: Nougyo-Gijutsu-Taikei Vegetables, Vol.11, Addendum No.34, 2009, Tokusan-Yasai 4-4 to 4-7, Rural Culture Association
Non-patent Literature 4: Agarie et al., Plant Prod. Sci. 12(1): 37-46 (2009), pp. 37-46

### Disclosure of Invention

### Problems to be solved by the Invention

The present invention focused on functional components contained in ice plant and aimed at producing functional materials derived from ice plant.

### Solutions to the Problems

The inventors of the present invention intensively studied, and as a result, surprisingly found that the contents of pinitol, β-carotene, vitamin K and proline which are functional components in an ice plant could be increased by addition of a stress during cultivation of ice plant. Thus, the present invention was completed.

That is, the present invention provides:
[1] A method of producing an ice plant having increased contents of pinitol, β-carotene, vitamin K and proline, which comprises adding a stress to an ice plant during cultivation of the ice plant;
[2] The method according to the above [1], wherein the cultivation is hydroponic cultivation;
[3] The method according to the above [1] or [2], wherein the stress is one or more stresses selected from the group consisting of the following (1) to (8):
   (1) change of pH,
   (2) elevation of temperature,
   (3) decrease of humidity,
   (4) ultraviolet irradiation,
   (5) increase of light intensity,
   (6) decrease of dissolved oxygen,
   (7) cutting of roots, and
   (8) increased level of potassium;
[4] The method according to any one of the above [1] to [3], whereby an ice plant further having a decreased content of nitrate nitrogen is obtained;
[5] The method according to any one of the above [1] to [4], wherein the obtained ice plant contains, per 100 g of plant fresh weight, 30 mg or more of pinitol, 1000 µg or more of β-carotene, 40 µg or more of vitamin K, and 7 mg or more of proline;
[6] The method according to any one of the above [1] to [5], wherein the obtained ice plant contains pinitol, β-carotene, vitamin K and proline at a ratio of 1 : 0.01 to 0.05 : 0.001 to 0.005 : 0.2 to 1.2;
[7] The method according to any one of [3] to [6], wherein the stress is one or more stresses selected from (1), (6) and (7);
[8] A method of increasing the contents of pinitol, β-carotene, vitamin K and proline in an ice plant, which comprises adding one or more stresses selected from the group consisting of the following (1) to (8) to an ice plant during cultivation of the ice plant:
   (1) change of pH,
   (2) elevation of temperature,
   (3) decrease of humidity,
   (4) ultraviolet irradiation,
   (5) increase of light intensity,
   (6) decrease of dissolved oxygen,
   (7) cutting of roots, and
   (8) increased level of potassium;
[9] The method according to [8], whereby the content of nitrate nitrogen in an ice plant is further decreased;
[10] An ice plant obtainable by the method according to any one of the above [1] to [9];
[11] An ice plant containing, per 100 g of plant fresh weight, 30 mg or more of pinitol, 1000 µg or more of β-carotene, 40 µg or more of vitamin K, and 7 mg or more of proline;
[12] The ice plant according to the above [11], which contains pinitol, β-carotene, vitamin K and proline at a ratio of 1 : 0.01 to 0.05 : 0.001 to 0.005 : 0.2 to 1.2;
[13] A natural functional material obtainable from the ice plant according to any one of the above [10] to [12];
[14] The natural functional material according to the above [13], which is in powderized form;
[15] A supplement containing the natural functional material according to the above [13] or [14]; and
[16] The supplement according to the above [15], which is a powder or a tablet.

### Effects of the Invention

According to the present invention, the contents of functional components pinitol, β-carotene, vitamin K and proline in an ice plant can be increased by addition of a stress during cultivation of ice plant, and an ice plant rich in such functional components can be efficiently obtained.

### Brief Description of Drawings

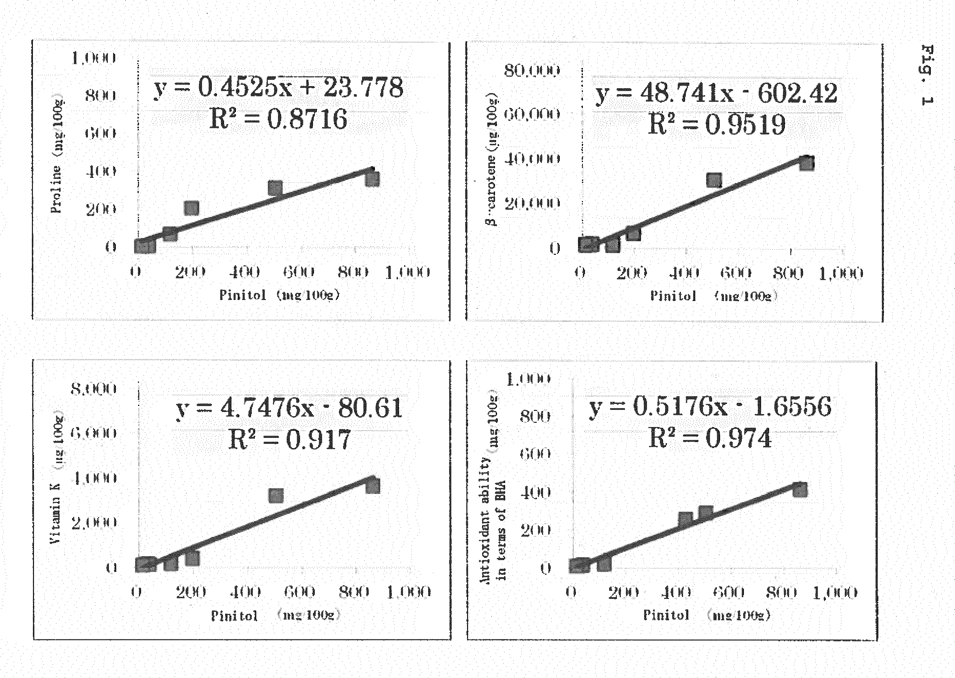

[Fig. 1] Graphs show correlations between pinitol and the other functional components contained a transition plant and a correlation between pinitol and antioxidant ability.

### Mode for Carrying Out the Invention

In the method of the present invention, any species of ice plant may be used.

In the method of the present invention, cultivation of an ice plant may be carried out by hydroponic cultivation, soil cultivation, or medium cultivation.

In the method of the present invention, a method of carrying out the hydroponic cultivation is not particularly limited, and the hydroponic cultivation may be carried out according to a general method for hydroponic cultivation. For example, ice plant seeds are sown on a water-retentive material, for example a urethane mat, and grown in a nursery box or the like until the 2-4 leaf stage. Then, the seedlings are transplanted to a hydroponic apparatus, and then grown. The material for sowing, the nursery box, the hydroponic apparatus, etc. which are used for the cultivation may be those generally used for hydroponic cultivation.

Usually, the hydroponic cultivation of an ice plant is carried out in a hydroponic solution containing no sodium chloride until the 4-5 leaf stage, and at the 6-7 leaf stage, about 50 mM sodium chloride is added to the hydroponic solution. The concentration of sodium chloride to be added to a hydroponic solution is appropriately adjusted so that a plant obtained can have a desired salty taste. As the hydroponic solution, a usual hydroponic solution for plant cultivation can be used, and for example, Otsuka House Fertilizer A or a 1/2 concentration-hydroponic solution thereof may be used. In the present invention, the hydroponic cultivation may be carried out according to a conventional method. It should be appreciated that the composition of a hydroponic solution, the concentration of sodium chloride to be added, timing of the addition, a cultivation period, etc. may be varied as appropriate. In the present invention, sodium chloride is not necessarily added.

In the method of the present invention, the soil cultivation may be carried out according to a general method for cultivation in a pot in a greenhouse. For cultivation of an ice , for example, seeds of an ice plant are sowed in a pot or a medium, and a nutrient solution prepared by diluting a liquid fertilizer, for example HYPONeX (registered trademark) (manufactured by HYPONeX JAPANA CORP., LTD.), to about 100 times is added into the pot or medium. A material for sowing, a nursery box, etc. which are used for the cultivation may be those generally used for soil cultivation.

The medium cultivation refers to a cultivation method using artificially prepared soil, for example smoked rice hulls, palm shells, peat moss, vermiculite, etc., and during the cultivation, a liquid fertilizer or the like is added as appropriate, in the same manner as soil cultivation. In the method of the present invention, the medium cultivation may be carried out using the above-described artificially prepared soil according to a conventional method.

In the method of the present invention, hydroponic cultivation is preferably used because it is easy to control conditions of stress in hydroponic cultivation.

In the method of the present invention, the cultivation of an ice plant is carried out in a greenhouse or a sunlight-combined type plant factory. Preferably, the cultivation of an ice plant in the present invention is carried out in an enclosed environment such as a room, for example, in a completely-controlled plant factory. Regardless of hydroponic cultivation, soil cultivation or medium cultivation, the ice plant cultivation in a plant factory has the advantages that ice plants can be stably produced all through the year without being affected by weather, can be safely produced without use of agricultural chemicals, and can be produced at a blank space or a farm land using an empty warehouse or a container.

The term "stress" as used herein means adverse conditions to growth of ice plant.

In the method of the present invention, the stress to be added to an ice plant may be any stress as long as it induces transition of the ice plant. Preferably, the stress is any stress other than salt stress. More preferably, the stress is one or more stresses selected from the group consisting of the following (1) to (8):
(1) change of pH,
(2) elevation of temperature,
(3) decrease of humidity,
(4) ultraviolet irradiation,
(5) increase of light intensity,
(6) decrease of dissolved oxygen,
(7) cutting of roots, and
(8) increased level of potassium.

Still more preferably, one or more stresses selected from the above-described (1) change of pH, (6) decrease of dissolved oxygen, and (7) cutting of roots are added. The addition of these stresses to an ice plant remarkably increases the contents of pinitol, β-carotene, vitamin K and proline in the ice plant. In particular, since the addition of these stresses can be easily controlled in hydroponic cultivation, these stresses are preferred.

In the method of the present invention, transition of an ice plant can be artificially induced by addition of a stress to the ice plant. The term "transition" as used herein means that the ice plant exhibits the same characteristics as those exhibited by an ice plant that has switched to CAM. For example, the ice plant that has undergone the "transition" according to the present invention exhibits increased contents of organic acids (malic acid, citric acid, etc.) in the plant body or change of the chlorophyll pigment to dark green.

Ice plant carries out C3 photosynthesis under suitable conditions. Ice plant is known to transition from C3 photosynthesis to CAM photosynthesis when it receives salt or drying stress or grows to a certain stage. The CAM photosynthesis is characterized by the fact that during the night, plants open their stomata to take up carbon dioxide and convert the carbon dioxide into malic acid to accumulate it, and during the day, they close the stomata, produce carbon dioxide from the malic acid accumulated during the night, and metabolize it. As used herein, the term "CAM-switching" means that an ice plant induces the expression of a group of enzymes required for CAM photosynthesis and gains a metabolic pathway (CAM pathway). The ice plant switching to CAM is characterized by increased contents of organic acids (malic acid, citric acid, etc.) in the plant body and slow growth. The ice plant switching to CAM is also characterized by change of the chlorophyll pigment to dark green.

The terms "ice plant that has undergone the transition" and "transition plant" as used herein mean ice plants exhibiting the same characteristics as those exhibited by the ice plant switching to CAM, including an ice plant that has switched to CAM and really carries out CAM photosynthesis, and an ice plant that has switched to CAM, but does not really carry on CAM photosynthesis. Therefore, the "ice plant that has undergone the transition" or the "transition plant" also includes an ice plant that exhibits the same characteristics as those exhibited by an ice plant switching to CAM, but really has not switched to CAM.

In the present invention, the "ice plant that has undergone the transition" or the "transition plant" can be selected by, for example, visual observation based on change of the appearance of a plant (hue of a plant) to dark green. Alternatively, the chlorophyll pigment content in a plant is measured by a chlorophyll meter (SPAD meter), and a plant having a higher measurement value than the measurement value of a non-stressed plant can be selected as the transition plant. Also, the transition plant may be selected based on measurement of an organic acid content (for example, the malic acid or citric acid content) in a plant.

In the method of the present invention, a stress may be added at any stage during a cultivation period of an ice plant. For example, a stress is added about 35-45 days, about 55-60 days or about 65-90 days after sowing seeds. An ice plant undergoes the transition about 5-15 days after the addition of a stress depending on the kind and condition of the stress.

In the present invention, a method of adding a stress to an ice plant is not particularly limited, and may be any method as long as it can accomplish the addition of the intended stress. A stress may be added gradually, intermittently, continuously, or at one time. Preferably, the addition of a stress at one time is effective depending on the kind of the stress. When one or more different kinds of stresses are added, they may be added at the same time or sequentially.

For the "change of pH" stress of the above-described (1), for example, the addition of the stress is carried out by decreasing or increasing the pH of a hydroponic solution with a pH adjusting agent or the like. The pH control is very important to hydroponic cultivation. When water and fertilizers are mixed to prepare a hydroponic solution, the pH of the hydroponic solution is usually controlled so as to become about 5.5-6.5. When the stress of the above-described (1) is added in the method of the present invention, for example, the pH of a hydroponic solution is increased or decreased by about 3. For example, the pH of a hydroponic solution is decreased to a pH of about 3.5-4.5, preferably about 3.0-4.0, more preferably about 2.5-3.5. When the pH of a hydroponic solution is decreased to a strongly acidic pH such as about pH 3, an ice plant undergoes the transition about 10 days after the addition of the stress. Alternatively, for example, the pH of a hydroponic solution is increased to a pH of about 7.5-8.5, preferably about 8.0-9.0, more preferably about 8.5-9.5. Examples of the pH adjusting agent that may be used include acidic substances usually used for pH adjustment, for example, phosphoric acid, sulfuric acid, nitric acid, citric acid, etc., and alkaline substances usually used for pH adjustment, for example, sodium hydroxide, potassium hydrogen carbonate, potassium hydroxide, coral comprising calcium carbonate as the mainly component, etc. Commercially available pH adjusting agents, for example, pH DOWN (manufactured by Otsuka Chemical Co., Ltd.) and pH UP (manufactured by Otsuka Chemical Co., Ltd.) may be also used.

For the "elevation of temperature" stress of the above-described (2), for example, the addition of the stress is carried out by elevating the temperature of a hydroponic solution (the temperature inside of a room) from about 20-23°C to about 25-28°C, preferably to about 27-30 °C, more preferably to about 28-32°C.

For the "decrease of humidity" stress of the above-described (3), for example, the addition of the stress is carried out by decreasing the humidity inside of a room under a closed environment to about 50-40%RH, preferably about 45-35%RH, more preferably about 40-30%RH. In the present invention, it has been found that an ice plant especially tends to undergo the transition at a humidity of 40%RH or below.

For the "ultraviolet irradiation" stress of the above-described (4), for example, the addition of the stress is carried out by exposure to ultraviolet irradiation at a wavelength of about 360-390 nm and an ultraviolet intensity of about 150-1000 uW/cm².

For the "increase of light intensity" stress of the above-described (5), for example, the addition of the stress is carried out by exposure to light irradiation at a strong light intensity of about 150-180 µmol/m²/second, preferably about 170-200 µmol/m²/second, more preferably about 200-250 µmol/m²/second for about 5-10 days, preferably for about 7-12 days, more preferably for about 9-15 days.

For the "decrease of dissolved oxygen" stress of the above-described (6), for example, the addition of the stress is carried out by stopping the circulation of a hydroponic solution. When the circulation of a hydroponic solution is stopped, oxygen dissolved in the hydroponic solution is decreased and plants become deficient in oxygen. For example, the stop of a hydroponic solution is carried out intermittently. For example, a pump for circulating a hydroponic solution is driven for about 0-23 hours, preferably for about 0-16 hours, more preferably for about 0-8 hours, and then stopped for about 1-24 hours, preferably for about 1-16 hours, more preferably for about 1-8 hours. The cycle of driving the pump and then stopping the pump as described above is repeated for about 1-40 days, preferably for about 1-30 days, more preferably for about 1-20 days. The lowest management value of dissolved oxygen level is usually about 5.5 mg/L. In the present invention, the stress is added so that the dissolved oxygen level in a hydroponic solution becomes for example about 5 mg/L or less, preferably about 4 mg/L or less, more preferably about 3 mg/L or less, still more preferably about 2 mg/L or less. In the present invention, it has been found that an ice plant especially tends to undergo the transition at a dissolved oxygen level of 4.0 mg/L or less. Also, oxygen dissolved in a hydroponic solution can be decreased by allowing plants to grow thickly and excessively and thereby inducing local stagnation of a nutrient solution.

For the "cutting of roots" stress of the above-described (7), for example, the addition of the stress is carried out by cutting the roots of an ice plant about 35-50 days, preferably about 50-60 days, more preferably about 50-55 days after sowing seeds.

For the "increased level of potassium" stress of the above-described (8), for example, the addition of the stress is carried out by adding potassium sulfate to a hydroponic solution at a concentration of 40-250 mM, preferably 40-170 mM, more preferably 90-170 mM. Potassium may be added instead of sodium chloride to be added to a hydroponic solution. Examples of a potassium source to be added to a hydroponic solution include Otsuka No. 10 (manufactured by Otsuka chemical Co., Ltd.) as well as potassium sulfate.

It is known that an ice plant accumulates functional components through the osmotic effect of sodium. However, when an ice plant is cultivated under high-salinity stress, the salt concentration absorbed into the ice plant is increased and therefore the ice plant has a very salty taste. In addition, such an ice plant is not preferred from the viewpoint of the health-conscious of limitation of salt intake. Therefore, it is very useful to produce the osmotic effect of potassium instead of sodium chloride. In the present invention, it has also been found that the use of potassium results in the addition of stronger stress than sodium.

When an ice plant undergoes the transition according to the method of the present invention, the contents of the functional components pinitol, β-carotene, vitamin K and proline in the ice plant are increased.

According to the method of the present invention, as compared with usual cultivation with no addition of stress, the content of pinitol in an ice plant is increased by for example at least about 1.5 times, preferably at least about 2.0 times, more preferably at least about 2.5 times, still more preferably at least about 3.0 times, the content of β-carotene in an ice plant is increased by at least about 1.2 times, for example at least about 1.5 times, preferably at least about 1.8 times, more preferably at least about 2.0 times, still more preferably at least about 2.5 times, the content of vitamin K in an ice plant is increased by at least about 1.2 times, for example at least about 1.5 times, preferably at least about 2.0 times, more preferably at least about 2.5 times, still more preferably at least about 3.0 times, and the content of proline in an ice plant is increased by at least about 3.0 times, preferably at least about 5.0 times, more preferably at least about 7.0 times, still more preferably at least about 9.0 times. The term "usual cultivation with no addition of stress" as used herein means that the cultivation is carried out by the same method as the method of the present invention except that the above-described stress for the method of the present invention is not added.

The ice plant that has undergone the transition by the method of the present invention contains, per 100 g of plant fresh weight, about 30 mg or more, for example about 60 mg or more, preferably about 100 mg or more, more preferably about 120 mg or more, more preferably about 150 mg or more of pinitol, about 1000 µg or more, for example about 1400 µg or more, preferably about 2500 µg or more, more preferably about 3500 µg or more, more preferably about 4000 µg or more of β-carotene, about 40 µg or more, for example about 120 µg or more, preferably about 200 µg or more, more preferably about 250 µg or more, more preferably about 300 µg or more of vitamin K, and about 7 mg or more, for example about 40 mg or more, preferably about 60 mg or more, more preferably about 80 mg or more, more preferably about 100 mg or more of proline. The content ratio by weight of pinitol, β-carotene, vitamin K and proline in the ice plant that has undergone the transition is preferably 1 : 0.01 to 0.05 : 0.001 to 0.005 : 0.2 to 1.2, more preferably 1 : 0.01 to 0.04 : 0.001 to 0.003 : 0.4 to 1.1, still more preferably 1 : 0.02 to 0.04 : 0.001 to 0.003 : 0.5 to 1.1.

In the present invention, it has further been found that when an ice plant undergoes the transition, the content of nitrate nitrogen in the ice plant is decreased. Nitrogen is important as a component of protein essential for plants and chlorophyll necessary for photosynthesis, and also contributes to absorption of nutrients, anabolism, and elongation of stems, leaves and roots. Plants preferably utilize nitrate nitrogen. However, when the anabolism process of the nitrate nitrogen absorbed into plants does not smoothly proceed, a large amount of nitrate accumulates in the plants. Since nitrate nitrogen oxidizes hemoglobin in blood to methemoglobin, some effects of nitrate nitrogen have been reported. For example, it has been reported that nitrate nitrogen causes hemoglobinemia particularly in infants, and leads to decreased availability of vitamin A in the body or decreased function of liver or thyroid gland. Therefore, it is preferable that the amount of nitrate in the plant body is decreased.

According to the method of the present invention, as compared with usual cultivation with no addition of stress, the amount of nitrate nitrogen in an ice plant is decreased by at least about 30%, for example at least about 40%, preferably at least about 50%, more preferably at least about 60%, more preferably at least about 70%, on the basis of the nitrate ion concentration in an ice plant. The ice plant that has undergone the transition by the method of the present invention contains nitrate nitrogen at a nitrate ion concentration of about 5000 ppm or less, preferably about 4000 ppm or less, more preferably about 3000 ppm or less, still more preferably about 2000 ppm or less.

According to the method of the present invention, an ice plant containing larger amounts of pinitol, β-carotene, vitamin K and proline than those of a usual ice plant can be obtained. According to the method of the present invention, an ice plant containing larger amounts of pinitol, β-carotene, vitamin K and proline than those of a usual ice plant and containing a smaller amount of nitrate nitrogen than that of a usual ice plant can be further obtained.

As another aspect of the present invention, an ice plant containing larger amounts of pinitol, β-carotene, vitamin K and proline than those of a usual ice plant is provided. In the present invention, the term "usual ice plant" means an ice plant cultivated with no addition of the above-described stress for the method of the present invention.

The ice plant of the present invention contains an at least about 1.5 times, preferably at least about 2.0 times, more preferably at least about 2.5 times, still more preferably at least about 3.0 times larger amount of pinitol; an at least about 1.2 times, for example at least about 1.5 times, preferably at least about 1.8 times, more preferably at least about 2.0 times, still more preferably at least about 2.5 times larger amount of β-carotene; an at least about 1.2 times, for example at least about 1.5 times, preferably at least about 2.0 times, more preferably at least about 2.5 times, still more preferably at least about 3.0 times larger amount of vitamin K; and an at least about 3 times, preferably at least about 5 times, more preferably at least about 7 times, still more preferably at least about 9 times larger amount of proline than those of the usual ice plant.

The ice plant of the present invention contains, per 100 g of plant fresh weight, about 30 mg or more, for example about 60 mg or more, preferably about 100 mg or more, more preferably about 120 mg or more, more preferably about 150 mg or more of pinitol, about 1000 µg or more, for example about 1400 µg or more, preferably about 2500 µg or more, more preferably about 3500 µg or more, more preferably about 4000 µg or more of β-carotene, about 40 µg or more, for example about 120 µg or more, preferably about 200 µg or more, more preferably about 250 µg or more, more preferably about 300 µg or more of vitamin K, and about 7 mg or more, for example about 40 mg or more, preferably about 60 mg or more, more preferably about 80 mg or more, more preferably about 100 mg or more of proline. The content ratio by weight of pinitol, β-carotene, vitamin K and proline in the ice plant of the present invention is preferably 1 : 0.01 to 0.05 : 0.001 to 0.005 : 0.2 to 1.2, more preferably 1 : 0.01 to 0.04 : 0.001 to 0.003 : 0.4 to 1.1, still more preferably 1 : 0.02 to 0.04 : 0.001 to 0.003 : 0.5 to 1.1.

The present invention further provides an ice plant containing larger amounts of pinitol, β-carotene, vitamin K and proline than those of the usual ice plant and containing a smaller amount of nitrate nitrogen than that of the usual ice plant. The amount of nitrate nitrogen in the ice plant of the present invention is decreased by at least about 30%, for example at least about 40%, preferably at least about 50%, more preferably at least about 60%, more preferably at least about 70%, as compared with the usual ice plant, on the basis of the nitrate ion concentration in an ice plant. The ice plant of the present invention contains nitrate nitrogen at a nitrate ion concentration of about 5000 ppm or less, preferably about 4000 ppm or less, more preferably about 3000 ppm or less, still more preferably about 2000 ppm or less.

Pinitol is a methoxy derivative of chiro-inositol and is known to have insulin-like blood-pressure lowering effect. Pinitol can make somatic cells sensitive to the effect of insulin, and thereby pinitol facilitates uptake of glucose into cells from blood at the time of insulin secretion. Although the study of pinitol is ongoing, it has been shown that scheduled administration of pinitol is effective for type II diabetes patients. Pinitol is probably useful for stabilization of a blood glucose level. β-carotene is an antioxidant active substance capable of scavenging oxygen radicals, and is also known to have anticancer effect. Vitamin K is an essential substance for normal blood coagulation, and is also necessary for fixing of calcium to bones. Proline is one of 20 important amino acids, and is known to have water retention effect. Proline is also necessary for collagen formation.

Since the ice plant of the present invention contains larger amounts of pinitol, β-carotene, vitamin K and proline which are functional components having useful effects as described above, the ice plant of the present invention is useful as a functional food. The ice plant of the present invention can be also used as a naturally-derived functional material by processing the ice plant of the present invention into an extract, a powder, an essence, etc.

A method of obtaining an extract or powder from the ice plant of the present invention may follow a conventional method. For example, the ice plant can be subjected to extraction with water or alcohol or by supercritical high-pressure technique. For example, the ice plant can be dried by vacuum-freeze drying, far-infrared ray drying, or hot-air drying, and then powderized. Examples of devices used for powderization include a vacuum-freeze dryer [(FD-15-FL) manufactured by NIHON TECHNO SERVICE CO., LTD.], a far-infrared ray food dryer [(V7513-S) manufactured by Vianove. Inc.], a constant temperature dryer [(NDO-410) manufactured by TOKYO RIKAKIKAI CO., LTD.], and a pulverizer [(WM-10) manufactured by Sansho Industry Co., Ltd.].

The functional material obtained from the ice plant of the present invention can be utilized as a material for various products, for example, supplements such as nutritional supplements and health supplements, drinks, pharmaceuticals, and cosmetics. The above-described products can be obtained in desired forms such as tablets, powder, liquid, capsules, cream, gels, aerosols, ointments, cataplasms, etc. by using excipients that are well known in the art, etc. as appropriate. For example, the functional material of the present invention can be granulated and mixed together with excipients, for example, crystalline cellulose, fatty acid ester, fine silicon dioxide, etc., and optionally other active ingredients, for example, soluble vegetable fiber, locust bean extract, edible yeast, various vitamins, etc., and then tableted. Since the ice plant of the present invention contains large amounts of useful functional components as describe above, products made using the functional material obtained from the ice plant of the present invention are effective for, for example, prevention of lifestyle diseases including diabetes, anti-aging, beauty, moisture retention, amelioration of polycystic ovarian syndrome, alleviation of indefinite complaint, recovery of fatigue, improvement of immunity, improvement of liver function, etc.

Hereinafter, the present invention is explained in detail by way of Examples to which the present invention is not limited.

### Example 1

### Cultivation method of plants and Confirmation method of transition

In all Examples, ice plants were cultivated by the following method.

Ice plant seeds were sown on Salad urethane (product name, manufactured by M Hydroponic Research Co., Ltd.) for hydroponic cultivation, sprouted in a nursery tray, and grown until the 2-4 leaf stage. Then, the plants were subjected to NFT circulation-type cultivation (Nutrient Film Technique circulation-type hydroponic cultivation) in a closed-type plant factory. A hydroponic solution of Otsuka formulation (containing nitrogen, phosphoric acid, potassium, lime, magnesium, manganese, boron, iron, copper, zinc, molybdenum, etc.) was used. Until each stress was added, the plants were grown under the conditions of pH 5.5-6.5, 24 hours of light, a cultivation density of 40.7 plants/m², an EC (electric conductivity) of 0.23-0.27, and a room temperature of 22°C. On the 15th day after sowing the seeds, sodium chloride wad added to the hydroponic solution at a concentration of 100 mM. However, in the present invention, the addition of sodium chloride is not necessarily needed..

The transition of ice plants was confirmed by visual observation of changes of the chlorophyll pigment of the stems and leaves to dark green. The chlorophyll pigment contents were measured by a chlorophyll meter (SPAD meter). As a result, normal plants that did not undergo the transition had a SPAD value of about 35-45, whereas transition plants had a SPAD value of 50 or more.

### Example 2

### Stress Test using Change of pH

Ice plants were cultivated as described in Example 1. On the 50th day after sowing the seeds, the pH of a hydroponic solution was increased or decreased by about 3 by addition of pH DOWN or pH UP to the hydroponic solution in an amount of 150 ml/1000 L. On the 10th day after the addition of the stress, the ice plants underwent the transition.

### Example 3

### Stress Test using Elevation of temperature

Ice plants were cultivated as described in Example 1. On the 55th day after sowing the seeds, the room temperature was elevated from 22°C to 30°C over about 40 hours. Here, the night temperature was also the same temperature. After the temperature elevation, the room temperature was returned to 22°C over 3 hours. On the 5th day after the addition of the stress, the ice plants underwent the transition.

### Example 4

### Stress Test using Decrease of humidity

Ice plants were cultivated as described in Example 1. On the 55th day after sowing the seeds, a nutrient solution within a cultivation bed was all discharged, and thereby roots became dry condition wherein they were exposed to air. As a result, on the 5th day after the addition of the stress, the ice plants underwent the transition.

### Example 5

### Stress Test using Ultraviolet irradiation

Ice plants were cultivated as described in Example 1. On the 55th day after sowing the seeds, ultraviolet irradiation (150-1000 uW/cm²) was started. As a result, on the 10th day after the addition of the stress, the ice plants underwent the transition.

### Example 6

### Stress Test using Increase of light intensity

Ice plants were sprouted and grown as described in Example 1. The seedlings were transplanted, and at the same time, they were exposed to strong light of 190 µmol/m²/s. As a result, on the 10th day after transplanting, all plants underwent the transition.

### Example 7

### Stress Test using Decrease of dissolved oxygen

Ice plants were cultivated as described in Example 1. From the 54th day after sowing the seeds, a nutrient solution was provided by repeating the cycle of "driving a pump for 1 hour and then stopping the pump for 3 hours". At this time, the concentration of dissolved oxygen in the solution was about 2 mg/L to 4 mg/L. On the 9th day after the addition of the stress, all plants underwent the transition.

### Example 8

### Stress Test using Cutting of roots

Ice plants were cultivated as described in Example 1. On the 50th, 55th, 56th or 60th day after sowing the seeds, the roots were cut. As a result, on the 10-15th day after the addition of the stress, the ice plants completely underwent the transition. In the case of test groups of the ice plants whose roots were cut on the 50th and 55th day after sowing the seeds, 100% of the ice plants underwent the transition. In the case of a test group of the ice plants whose roots were cut on the 56th day after sowing the seeds, 89% of the ice plants underwent the transition. On the other hand, in the case of a test group of the ice plants whose roots were cut on the 60th day after sowing the seeds, 100% of the ice plants did not undergo the transition.

### Example 9

### Stress Test using Increased level of potassium

Ice plants were cultivated as described in Example 1 except the concentration of sodium chloride added to a hydroponic solution. Instead of sodium chloride, the equal molar concentration of potassium sulfate was added to a hydroponic solution. As controls, experiments were performed with a hydroponic solution not containing sodium chloride and potassium sulfate, and with a hydroponic solution not containing the equal molar concentration of sodium chloride. Each experimental group comprised 6 ice plants. Forty days after sowing the seeds, 86 mM (0.5%) sodium chloride, 171 mM (1%) sodium chloride, 342 mM (2%) sodium chloride, 513 mM (3%) sodium chloride, 43 mM potassium sulfate, 85.5 mM potassium sulfate, 171 mM potassium sulfate, or 256.5 mM potassium sulfate was added to the hydroponic solution.

As a result, in a group with the addition of 342 mM of sodium chloride, on the 15th day after the addition, 40% of the ice plants underwent the transition. On the other hand, in a group with the addition of 171 mM potassium sulfate, on the 15th day after the addition, 90% of the ice plants underwent the transition.

### Example 10

### Functional component analytical experiment 1

Ice plants were cultivated as described in Example 1. On the 55th day after sowing the seeds, the roots were cut. On the 10 day after the roots were cut, the plants that underwent the transition were harvested. These plants were called "transition plant A".

Ice plants were cultivated as described in Example 1 except that sodium chloride was not added to a hydroponic solution. On the 55th day after sowing the seeds, the roots were cut. On the 10 day after the roots were cut, the plants that underwent the transition were harvested. These plants were called "transition plant B".

As a control, ice plants were cultivated as described in Example 1, and on the 65th day after sowing the seeds, the plants were harvested. These plants were called "control plant".

The transition plant A, transition plant B and control plant thus harvested were subjected to the following functional component analyses.

### Pinitol analysis

Pinitol analysis was carried out using high-performance liquid chromatography (HPLC) (Simadzu Corporation, detector: differential refractometer detector). A sample solution was injected into a ShodexDC-613 column (6.0 mm I.D. x 150 mm L) through which acetonitrile/water = 75/25 (V/V) as a mobile phase was run at a flow rate of 1.0 mL/min and at 70°C, and then pinitol was detected. Three individuals for each of the transition plant A, the transition plant B and the control plant were subjected to the analysis. Specifically, the analysis was carried out by the following procedure. A suitable amount of the whole plant was ground with pure water by a homogenizer, centrifuged at 10000 rpm, and then filtered. A filtrate thus obtained was adjusted to a constant volume, and then filtered with a 0.20 µm membrane filter to obtain a sample solution.

### Proline analysis

Proline analysis was carried out following a method of Bates [Bates, L.S., R.P. Waldren, I.D. Teare, Rapid determination of free proline for water-stress studies, Plant and Soil 39, 205-207 (1973)]. Briefly, the whole plant was ground with 3% (W/V) sulfosalicylic acid and centrifuged to obtain a sample extract. To 2 mL of the sample extract, 2 mL of an acid ninhydrin solution and 2 mL of glacial acetic acid were added. They were reacted at **100°C** for 1 hour. After 4 mL of toluene was added, the reaction solution was strongly stirred and then an absorbance was measured at 520 nm.

### β-Carotene analysis

β-Carotene analysis was carried out using high-performance liquid chromatography (HPLC) (Hitachi High-Technologies Corporation, detector: L-2455 type diode-array detector). A sample solution was injected into a Hitachi LaChromCl8 column (4.6 mm I.D. x 150 mm L) through which methanol/ethanol = 5/1 (V/V) as a mobile phase was run at a flow rate of 0.8 mL/min and at 40°C, and then β-carotene was detected at a wavelength of 455 nm. Three individuals for each of the transition plant A, the transition plant B and the control plant were subjected to the analysis. Specifically, the analysis was carried out by the following procedure. To a suitable amount of the whole plant, 5 mL of 3% (W/V) pyrogallol-ethanol and 0.5 mL of **60**% (W/V) potassium hydroxide were added, followed by saponification at 70°C for 30 minutes. After water-cooling and addition of 11.25 mL of 1% (W/V) sodium chloride, an extraction operation with 7.5 mL of a mixture (9:1 V/V) of hexane-ethyl acetate by shaking and centrifugation was repeated three times. An extract thus obtained was concentrated under reduced pressure at 40°C. A residue was dissolved in a constant amount of chloroform, and then filtered with a 0.20 µm membrane. A filtrate was used as a sample solution.

### Vitamin K analysis

Vitamin K analysis was carried out using high-performance liquid chromatography (Hitachi High-Technologies Corporation, detector: L-2455 type diode-array detector). A sample solution was injected into a Hitachi LaChromC18 column (4.6 mm I.D. x 150 mm L) through which acetonitrile/methanol = 60/40 (V/V) as a mobile phase was run at a flow rate of 1.0 mL/min and at 40°C, and then vitamin K was detected at a wavelength of 265 nm. Three individuals for each of the transition plant A, the transition plant B and the control plant were subjected to the analysis. Specifically, the analysis was carried out by the following procedure. A suitable amount of the whole plant was treated with 5 mL of a 1% (W/V) citric acid solution at 60°C for 5 minutes, adjusted to a constant volume with acetone, placed in a ultrasonic bath for 10 minutes, and allowed to still stand overnight to obtain an extract. To 5 mL of the extract thus obtained, 5 mL of ethanol and 5 mL of a 1% (W/V) citric acid solution were added. Then, an extraction operation with 7.5 mL of a mixture (9:1 V/V) of hexane-ethyl acetate by shaking and centrifugation was repeated three times. An extract thus obtained was concentrated under reduced pressure at 40°C. A residue was dissolved in 5 mL of hexane, and then applied to a silica gel-packing mini column. Then, vitamin K retained in the column was eluted with 30 mL of hexane-diethyl ether (85:15 V/V). An eluate was concentrated under reduced pressure at 40°C. A residue was dissolved in a constant amount of methanol and filtered with a 0.20 µm membrane. A filtrate was used as a sample solution.

### Malic acid analysis

Malic acid analysis was carried out using high-performance liquid chromatography [Hitachi High-Technologies Corporation, Organic acid (BTB method) Analysis System, detector: L-2420 type UV-VIS detector]. A sample solution was injected into a Hitachi GL-C610H-S column (7.8 mm I.D. x 300 mm L) through which 3 mmol/L perchloric acid as a mobile phase was run at a flow rate of 0.5 mL/min and at 40°C. The column was separated and then reacted with a BTB solution, and malic acid was detected at a wavelength of 440 nm. Three individuals for each of the transition plant A, the transition plant B and the control plant were subjected to the analysis. Specifically, the analysis was carried out by the following procedure. A suitable amount of the whole plant was ground with pure water by a homogenizer, centrifuged at 10000 rpm, and filtered. A filtrate thus obtained was adjusted to a constant volume, and then filtered with a 0.20 µm membrane filter to obtain a sample solution.

### Antioxidant ability analysis

Further, the antioxidant ability of an ice plant was analyzed. As used herein, the antioxidant ability means the function of antioxidant components including β-carotene. The antioxidant components include, in addition to β-carotene, betacyanin, vitamin E, vitamin C, polyphenol, etc.

Antioxidant ability analysis was carried out following a method of Ikeba et al. [Tomoko Ikeba, and Kyoko Kashima, "Evaluation and change by cooking of the antioxidative property of vegetables produced in Ibaraki prefecture", Research report No. 14, 27-33 (2006), Agricultural Research Institute, Ibaraki Agricultural Center]. Briefly, a sample was ground with 80% ethanol, and thereto a linoleic acid-β carotene solution was added. The sample was quickly placed in a thermostatic bath at 50°C. After 15 minutes and 45 minutes, an absorbance A (wavelength: 470 nm) was measured to calculate a value of ΔA = (A 15 minutes) - (A 45 minutes). There is a linear relation between a value of ΔA and a logarithmic value of the concentration of butylhydroxyanisole (hereinafter, referred to as BHA) which is a synthetic antioxidant. Utilizing this relation, ΔA was determined using a BHA standard solution and a standard curve was made. The antioxidative property of a sample solution was evaluated in terms of the corresponding BHA concentration based on the standard curve.

Results are shown in Table 1.

**[Table 1]**

| Table 1. Measurement data of control plant and transition plant of fresh ice plant | | | | | | |
|---|---|---|---|---|---|---|
| | Control plant | Component ratio | Transition plant A | Component ratio | Transition plant B | Component ratio |
| Pinitiol (mg/100g FW) | 32 | 1 | 117 | 1 | 196 | 1 |
| Proline (mg/100g FW) | 8 | 0.26 | 68 | 0.57 | 206 | 1.0 |
| β-carotene (µg/100g FW) | 1,592 | 0.050 | 1,456 | 0.012 | 6,556 | 0.033 |
| Vitamin K (µg/100g FW) | 155 | 0.0049 | 164 | 0.0014 | 411 | 0.0021 |
| Malic acid (mg/100g FW) | 3 | | 140 | | 380 | |
| Antioxidant ability in terms of BHA (mg/100g FW) | 13 | | 21 | | 30 | |

| | | | | | | |
|---|---|---|---|---|---|---|
| Transition plant A: Addition of salt + Cutting of roots Transition plant B: No addition of salt + Cutting of roots FW means Fresh Weight. | | | | | | |

Under the salt stress, much energy is probably expended on osmotic adjustment within cells in order to eliminate the strong toxicity of Na. Thus, in the transition plant A, only pinitol and proline were increased because the production of pinitol and proline, which are osmotic adjusters, probably got preference over the production of substances such as β-carotene and vitamin K. In addition, the substance production was probably inhibited by high Na concentration within the cells. On the other hand, in the transition plant B which was not subjected to the salt stress, β-carotene and vitamin K were increased because adequate energy could be probably expended to respond to the stress caused by root cutting (for example, to produce antioxidative substances) and there was little inhibition of enzymatic reaction by salt. At this time, the reason why pinitol and proline were also increased in spite of no stress of salt is probably that there was osmotic stress or the like associated with wilt.

Further, the content of each functional component in an analysis sample was plotted on a vertical axis and the content of pinitol in the analysis sample was plotted on a horizontal axis, thereby a correlation factor was calculated. As a result, as shown in Figure 1, it was found that there was a correlation between pinitol and each functional component contained in the transition plant.

### Example 11

### Measurement of nitrate nitrogen

Measurement of nitrate nitrogen in an ice plant was carried out using a compact nitrate ion meter B-341 manufactured by HORIBA. Briefly, the whole ice plant was squeezed by a squeezer, and a homogenized sample was dropped on a measurement sensor to measure the nitrate nitrogen.

The transition plant A and the control plant as described in Example 10 were subjected to the measurement of nitrate nitrogen. As a result, the nitrate nitrogen content in the transition plant was a nitrate ion concentration of 1,900 ppm. On the other hand, the nitrate nitrogen content in the control plant was a nitrate ion concentration of 2,800 ppm. Thus, the nitrate nitrogen content in the transition plant was decreased by about 32% as compared with the control plant.

### Example 12

### Production of functional material 1

As ice plant raw materials, the transition plant A, transition plant B and control plant obtained according to Example 10 were dried by a vacuum-freeze dryer, a hot-air dryer or a far-infrared ray dryer, and then powderized by a pulverizer to obtain powder with a particle size of 200 µm or less. A yield rate from the fresh weight to the powder was about 3-5%. The water content of the powder was adjusted to less than 5%. As the dryer, a vacuum-freeze dryer FD-15-FL (manufactured by NIHON TECHNO SERVICE CO., LTD.), a far-infrared ray food dryer V7513-S (manufactured by Vianove. Inc.), or a constant temperature dryer NDO-410 (manufactured by TOKYO RIKAKIKAI CO., LTD.) was used. As the pulverizer, a pulverizer WM-10 (manufactured by Sansho Industry Co., Ltd.) was used.

### Functional component analysis

The ice plant freeze-dried powder as obtained by the above-described method was subjected to functional component analysis in the same manner as Example 10.

Results are shown in Table 2.

**[Table 2]**

| Table 2. Measurement data of freeze-dried powder of control plant and freeze-dried powder of transition plant | | | |
|---|---|---|---|
| | Control plant powder | Transition plant A powder | Transition plant B powder |
| Pinitiol (mg/100g DW) | 862 | 1,462 | 2,590 |
| Proline (mg/100g DW) | 361 | 1,377 | 2,900 |
| β-carotene (µg/100g DW) | 38,546 | 34,792 | 46,337 |
| Vitamin K (µg/100g DW) | 3,630 | 3,710 | 4,787 |
| Malic acid (mg/100g DW) | 58 | 2,160 | 3,566 |
| Antioxidant ability in terms of BHA (mg/100g DW) | 415 | 482 | 521 |

| | | | |
|---|---|---|---|
| Transition plant A: Addition of salt + Cutting of roots Transition plant B: No addition of salt + Cutting of roots DW means Dry Weight. | | | |

### Measurement of nitrate nitrogen

The ice plant freeze-dried powder of the transition plant A and the ice plant freeze-dried powder of the control plant as obtained by the above-described method were subjected to measurement of nitrate nitrogen in the same manner as Example 11.

The nitrate nitrogen content in the transition plant powder was a nitrate ion concentration of 34,000 ppm. On the other hand, the nitrate nitrogen content in the control plant powder was a nitrate ion concentration of 120,000 ppm. Thus, the nitrate nitrogen content in the transition plant was decreased by about 72% as compared with the control plant.

### Example 13

### Functional component analytical experiment 2

Ice plants were cultivated as described in Example 1 except that sodium chloride was not added to a hydroponic solution. From the 54th day after sowing the seeds, a nutrient solution was provided by repeating the cycle of "driving a pump for 1 hour and then stopping the pump for 3 hours". At this time, the concentration of dissolved oxygen in the solution was about 2 mg/L to 4 mg/L. On the 9th day after the addition of the stress, the plants that underwent the transition were harvested. These plants were called "transition plant C".

Ice plants were cultivated as described in Example 1 except that sodium chloride was not added to a hydroponic solution. On the 50th day after sowing the seeds, the pH of the hydroponic solution was changed from 6.1 to 3.2 by addition of pH DOWN to the hydroponic solution in an amount of 150 ml/1000 L. On the 10th day after the addition of the stress, the plants that underwent the transition were harvested. These plants were called "transition plant D".

The transition plant C and transition plant D thus harvested were subjected to functional component analyses in the same manner as Example 10.

Results are shown in Table 3.

**[Table 3]**

| Table 3. Measurement data of control plant and transition plant of fresh ice plant | | | | | | |
|---|---|---|---|---|---|---|
| | Control plant | Component ratio | Transition plant C | Component ratio | Transition plant D | Component ratio |
| Pinitiol (mg/100g FW) | 32 | 1 | 202 | 1 | 105 | 1 |
| Proline (mg/100g FW) | 9 | 0.26 | 162 | 0.80 | 47 | 0.45 |
| β-carotene (µg/100g FW) | 1,592 | 0.050 | 4,895 | 0.024 | 1,918 | 0.018 |
| Vitamin K (µg/100g FW) | 155 | 0.0049 | 310 | 0.0015 | 212 | 0.0020 |
| Malic acid (mg/100g FW) | 3 | | 241 | | 177 | |
| Antioxidant ability in terms of BHA (mg/100g FW) | 13 | | 28 | | 22 | |

| | | | | | | |
|---|---|---|---|---|---|---|
| Transition plant C: No addition of salt + decrease of dissolved oxygen Transition plant D: No addition of salt + change of pH FW means Fresh Weight. | | | | | | |

### Example 14

### Production of functional material 2

As ice plant raw materials, the transition plant C and the transition plant D obtained according to Example 13 were powderized in the same manner as Example 12. A yield rate from the fresh weight to the powder was about 3-5%. The water content of the powder was adjusted to less than 5%.

### Functional component analysis

The ice plant freeze-dried powder as obtained by the above-described method was subjected to functional component analyses in the same manner as Example 10. The control plant was the same as described in Example 12.

Results are shown in Table 4.

**[Table 4]**

| Table 4. Measurement data of freeze-dried powder of control plant and freeze-dried powder of transition plant | | | |
|---|---|---|---|
| | Control plant powder | Transition plant C powder | Transition plant D powder |
| Pinitiol (mg/100g DW) | 862 | 2,916 | 1,450 |
| Proline (mg/100g DW) | 361 | 2,265 | 1,543 |
| β-carotene (µg/100g DW) | 38,546 | 43,499 | 39,097 |
| Vitamin K (µg/100g DW) | 3,630 | 4,545 | 4,008 |
| Malic acid (mg/100g DW) | 58 | 3,153 | 2,722 |
| Antioxidant ability in terms of BHA (mg/100g DW) | 415 | 533 | 504 |

| | | | |
|---|---|---|---|
| Transition plant C: No addition of salt + decrease of dissolved oxygen Transition plant D: No addition of salt + change of pH DW means Dry Weight. | | | |

### Example 15

### Production of supplement

Ice plant powder was produced according to Example 12 or Example 14. The ice plant powder was granulated and mixed together with soluble vegetable fiber, edible yeast, locust bean extract, vitamins, crystalline cellulose, fatty acid ester, and fine silicon dioxide, and then tableted, by a conventional method, to produce a supplement comprising naturally-derived functional components.

### Industrial Applicability

According to the present invention, the contents of functional components pinitol, β-carotene, vitamin K and proline in an ice plant can be increased by addition of a stress during cultivation of the ice plant, and an ice plant rich in such functional components can be efficiently obtained. Such an ice plant rich in functional components can be used as a natural functional material in a broad range of fields, for example in the fields of food products, pharmaceuticals, cosmetics, etc.

## Claims

1. A method of producing an ice plant having increased contents of pinitol, β-carotene, vitamin K and proline, which comprises adding a stress to an ice plant during cultivation of the ice plant.

2. The method according to claim 1, wherein the cultivation is hydroponic cultivation.

3. The method according to claim 1 or 2, wherein the stress is one or more stresses selected from the group consisting of the following (1) to (8):
(1) change of pH,
(2) elevation of temperature,
(3) decrease of humidity,
(4) ultraviolet irradiation,
(5) increase of light intensity,
(6) decrease of dissolved oxygen,
(7) cutting of roots, and
(8) increased level of potassium.

4. The method according to any one of claims 1 to 3, whereby an ice plant further having a decreased content of nitrate nitrogen is obtained.

5. The method according to any one of claims 1 to 4, wherein the obtained ice plant contains, per 100 g of plant fresh weight, 30 mg or more of pinitol, 1000 µg or more of β-carotene, 40 µg or more of vitamin K, and 7 mg or more of proline.

6. The method according to any one of claims 1 to 5, wherein the obtained ice plant contains pinitol, β-carotene, vitamin K and proline at a ratio of 1 : 0.01 to 0.05 : 0.001 to 0.005 : 0.2 to 1.2.

7. The method according to any one of claims 3 to 6, wherein the stress is one or more stresses selected from (1), (6) and (7).

8. A method of increasing the contents of pinitol, β-carotene, vitamin K and proline in an ice plant, which comprises adding one or more stresses selected from the group consisting of the following (1) to (8) to an ice plant during cultivation of the ice plant:
(1) change of pH,
(2) elevation of temperature,
(3) decrease of humidity,
(4) ultraviolet irradiation,
(5) increase of light intensity,
(6) decrease of dissolved oxygen,
(7) cutting of roots, and
(8) increased level of potassium.

9. The method according to claim 8, whereby the content of nitrate nitrogen in an ice plant is further decreased.

10. An ice plant obtainable by the method according to any one of claims 1 to 9.

11. An ice plant containing, per 100 g of plant fresh weight, 30 mg or more of pinitol, 1000 µg or more of β-carotene, 40 µg or more of vitamin K, and 7 mg or more of proline.

12. The ice plant according to claim 11, which contains pinitol, β-carotene, vitamin K and proline at a ratio of 1 : 0.01 to 0.05 : 0.001 to 0.005 : 0.2 to 1.2.

13. A natural functional material obtainable from the ice plant according to any one of claims 10 to 12.

14. The natural functional material according to claim 13, which is in powderized form.

15. A supplement containing the natural functional material according to claim 13 or 14.

16. The supplement according to claim 15, which is a powder or a tablet.
